# EUROPÄISCHE PATENTSCHRIFT

(11) **EP 3 213 376 B1**
(45) Veröffentlichungstag und Bekanntmachung des Hinweises auf die Patenterteilung: **26.09.2018**
(21) Anmeldenummer: 15828332.5
(22) Anmeldetag: 30.10.2015
(51) Int. Cl.: H01R 13/52, A61N 1/375, H01R 13/22, H01R 13/645, H01R 13/639, A61N 1/05, H01R 24/84

(54) **IMPLANTIERBARER STECKVERBINDER**
IMPLANTABLE PLUG CONNECTOR
CONNECTEUR IMPLANTABLE

(30) Priorität: 30.10.2014 DE 102014115859
(43) Veröffentlichungstag der Anmeldung: 06.09.2017
(73) Patentinhaber: CorTec GmbH, 79110 Freiburg (DE)
(72) Erfinder: SCHÜTTLER, Martin, 79312 Emmendingen (DE); KOCH, Julia, 79100 Freiburg (DE); ORDONEZ, Juan Sebastian, 79106 Freiburg (DE); RICKERT, Jörn, 79104 Freiburg (DE)
(74) Vertreter: 2s-ip Schramm Schneider Bertagnoll Patent- und Rechtsanwälte Part mbB
(86) Internationale Anmeldenummer: PCT/EP2015/075286
(87) Internationale Veröffentlichungsnummer: WO 2016/066815

(56) Entgegenhaltungen:
- WO-A1-2013/048396
- DE-A1- 19 622 669
- US-A1- 2005 118 887
- US-A1- 2010 042 169
- US-B1- 6 321 126
- US-B1- 7 445 528

## Beschreibung

Die vorliegende Erfindung bezieht auf einen implantierbaren Steckverbinder.

Aktive Implantate (Active Implantable Medical Devices = AIMDs) haben sich fest in den modernen medizinischen Therapiemöglichkeiten etabliert. Meist setzen sich aktive Implantate aus den zwei, über Kabel miteinander verknüpften Grundmodulen Stimulationseinheit und Elektroden zusammen. Diese liegen bisweilen weit auseinander, da am unmittelbaren Wirkungsbereich des Implantats nicht genügend Platz für den Stimulator ist.

So wird z.B. bei Neurostimulatoren der Stimulator im Brustbereich in einer Hautfalte platziert; Kabel von der Brust über den Nacken bis an den Schädel verbinden ihn mit den Elektroden im Gehirn. Während frühe AIMD-Systeme sozusagen "aus einem Guss" bestanden, also Stimulationseinheit, Kabel und Elektroden fest miteinander verbunden waren, haben sich mittlerweile mehrteilige Bauweisen durchgesetzt. Die Teile werden zunächst einzeln implantiert. Die elektrische Verbindung der Einzelteile erfolgt anschließend. Hierzu bedarf es eines implantierbaren Steckverbinders. Dieser kann, wie bei Herzschrittmachern üblich, direkt am Gehäuse des Stimulators oder wie beispielweise bei Neuroprothesen im Kabelbereich zwischen den Komponenten sitzen. Letzteres findet man in der Praxis allerdings nur für bis zu vier Kanäle und somit sehr "schlanken" Steckverbindungen, höherkanalige Varianten hingegen sitzen direkt am Stimulator.

Weiterentwicklungen führen zu immer kleineren Systemen mit komplexeren Stimulations- und Detektionsfunktionen. Dies geht eng einher mit einer stetigen Erhöhung der Kanalzahl zur selektiveren Stimulation. Hiermit steigen auch die Anforderungen an den Steckverbinder, welcher eine Weiterleitung der Signale gewährleistet.

Bekannte elektrische Steckverbinder sind nach dem Stecker-Steckdose-Prinzip ("Männchen"-Weibchen"-Prinzip) ausgestaltet. Hierbei ist der Männchen-Teil durch einen oder mehrere Stifte gebildet, die im Montage-Zustand im Weibchen-Teil in entsprechende Buchsen eingeführt sind. Die Stifte des Männchen-Teils stehen also über die Oberfläche, welche die Stifte trägt, hervor. An den Stiften und an den Buchsen können sich bei der Implantation im Vormontage-Zustand Verschmutzungen anlagern.

US 6 321 126 B1 betrifft einen implantierbaren Steckverbinder gemäß dem Oberbegriff des Anspruchs 1.

US 2005/118887 A1 sowie DE19622669 A1 betreffen jeweils einen implantierbaren Steckverbinder, bei welchem hervorstehende Kontaktflächen sich versenkten Kontaktflächen gegenüber befinden.

Aufgabe ist es, einen implantierbaren Steckverbinder zu schaffen, der eine vereinfachte Steckerkonstruktion hat.

Diese Aufgabe wird durch einen implantierbaren Steckverbinder gemäß Anspruch 1 und ein Herstellungsverfahren gemäß dem unabhängigen Verfahrensanspruch gelöst. Vorteilhafte Weiterbildungen sind in den abhängigen Ansprüchen definiert.

Erfindungsgemäß bereitgestellt wird also ein implantierbarer Steckverbinder, aufweisend:
einen Innenabschnitt, umfassend eine Anzahl von Kontaktflächen, die in zumindest einer Oberfläche eines ersten Substrats eingebettet sind,
einen Außenabschnitt, umfassend eine Anzahl von Kontaktflächen, die in zumindest einer Oberfläche eines zweiten Substrats eingebettet sind,
wobei der Außenabschnitt einen Raum definiert, in welchem der Innenabschnitt in einem Montage-Zustand aufnehmbar ist,
wobei die Kontaktflächen in einem Vormontage-Zustand im Verhältnis zu der Oberfläche des jeweiligen Substrats zurückgesetzt sind,
und wobei in einem Montage-Zustand der Innenabschnitt gegen den Außenabschnitt gepresst ist, derart, dass zueinander korrespondierende Kontaktflächen miteinander in Berührung kommen.

Weil die Kontaktflächen gegenüber der Oberfläche, in der sie eingebettet sind, zurückgesetzt sind, kann die Verschmutzung der Kontakte bei der Implantation, wie sie bei den bekannten Steckkontakten üblich sind, besser vermieden werden. Es handelt sich also um eine "Weibchen-Weibchen"-Verbindung, denn beide einander entsprechenden Kontakte sind, im Vormontage-Zustand, jeweils im Verhältnis zur Oberfläche, in der sie liegen, zurückgesetzt. Sie befinden sich also in Vertiefungen innerhalb des jeweiligen Substrats. Bei der Montage werden der Innenabschnitt und der Außenabschnitt derart aufeinander gepresst, dass die Kontaktflächen an die Oberfläche kommen und sich dann einander gegenüberliegende Kontaktflächen paarweise berühren und der Steckverbinder somit elektrisch leitend wird.

Der Innenabschnitt bildet also den einen Teil des implantierbaren Steckverbinders, und der Außenabschnitt bildet den anderen Teil des implantierbaren Steckverbinders. Im Montagezustand sind beide Teile zusammengefügt.

Der Innenabschnitt beinhaltet also das erste Substrat, und der Außenabschnitt beinhaltet also das zweite Substrat.

Die Aufeinanderpressung der beiden Abschnitte kann so erfolgen, dass benachbarte Kontaktflächen gegeneinander elektrisch isoliert sind.

Wenn die Kontaktflächen zumindest stellenweise strukturiert sind, wird die Kontaktierung im Montage-Zustand sicherer gewährleistet. Als besonders vorteilhaft haben sich kammartige, konvexe Ausbuchtungen erwiesen.

Durch die Aufeinanderpressung der beiden Abschnitte im Montage-Zustand können die Kontaktflächen gegen den Raum abgedichtet sein. Dies heißt, dass die Kontaktflächen nicht in Berührung mit Körperflüssigkeiten aus der Umgebung kommen.

Der Außenabschnitt kann zumindest zwei Gehäuseteile mit jeweils vorbestimmten Tiefen umfassen, die im Montage-Zustand den Raum definieren. Die Gehäuseteile können symmetrisch zueinander sein, es kann sich also um Gehäusehälften (Halbschalen) handeln.

Zumindest einer der Gehäuseteile, vorzugsweise beide Gehäuseteile des Außenabschnitts können hierbei jeweils eine Oberfläche mit eingebetteten Kontaktflächen umfassen.

Im Vormontage-Zustand kann die Summe der Tiefen der Gehäuseteile geringer als die Höhe des Innenabschnitts sein. Dann ist am Einfachsten gewährleistet, dass beim Aneinanderfügen der Gehäuseteile Druck auf das Innenteil ausgeübt wird und die Kontaktflächen miteinander in Berührung kommen.

Der Steckverbinder kann weiter aufweisen: Eine Schraubverbindung, mit welcher im Montage-Zustand der Innenabschnitt gegen den Außenabschnitt gepresst ist. Eine Schraubverbindung (Schraube und Gewindeloch) ist besonders gut geeignet - wenn auch nicht die einzige Möglichkeit -, im Montage-Zustand den Anpressdruck einzustellen.

Der Steckverbinder kann des Weiteren einen Kanal in Axialrichtung aufweisen, der ein Einführen eines Führungsdrahts ermöglicht. Die Axialrichtung ist eine gedachte Linie zwischen denjenigen Punkten gemeint, an denen die Kabel die jeweiligen Enden der Steckverbinder durchdringen. Der Führungsdraht wird benötigt, um die jeweiligen Teile bei der Operation jeweils in den menschlichen (oder tierischen) Körper einzuführen.

Um zu verhindern, dass der Führungsdraht im Körper vergessen wird, kann vorgesehen sein, dass der Kanal im Montage-Zustand durch die Schraubverbindung blockiert ist, die Schrauben sich also nicht eindrehen lassen, ohne dass der Führungsdraht zuvor entfernt wurde.

Des Weiteren können Kontaktzonen vorgesehen sein, die einerseits mit Leitungen andererseits mit den Kontaktflächen verbindbar sind.

Des Weiteren können Verpolungssicherungsmittel vorgesehen sein, welche im Montage-Zustand gewährleisten, dass der Innenabschnitt in genau einer Orientierung bezüglich des Außenabschnitts in dem Außenabschnitt aufnehmbar ist. Dies schafft eine eindeutige relative Orientierung von Innenteil und Außenteil und damit eine eindeutige Zuordnung der Kontaktfläche zueinander.

Der Außenabschnitt und/oder der Innenabschnitt können mit Silikon vergossen sein.

Als Material für den Außenabschnitt oder Teile davon kommt insbesondere Metall, vorzugsweise Titan zum Einsatz.

Die Erfindung umfasst auch ein Verfahren zum Herstellen von Kontaktflächen für einen Steckverbinder, insbesondere für einen Steckverbinder gemäß der Erfindung, umfassend die Schritte:
- Aufschleudern einer ersten Silikonschicht auf ein Keramiksubstrat;
- Aufwalzen von Metall;
- Strukturieren des aufgewalzten Metalls zur Bildung von Leiterbahnen;
- Aufschleudern einer zweiten Silikonschicht;
- Freilegen der Kontaktflächen.

Das Strukturieren erfolgt vorzugsweise mittels eines Pikosenkundenlasers.

Die freigelegten Kontaktflächen können strukturiert werden, vorzugsweise mittels eines Nanoseklundenlasers.

Die Erfindung wird anhand von Ausführungsbeispielen und der Zeichnung näher beschrieben. In der Zeichnung zeigt:
Fig. 1 ein Ausführungsbeispiel des Steckverbinders gemäß der Erfindung im V ormontage-Zustand,
Fig. 2 den Steckverbinder des Ausführungsbeispiels aus einer anderen Perspektive,
Fig. 3 den Steckverbinder im Montage-Zustand,
Fig. 4 den Steckverbinder im Montage-Zustand in Seitenansicht,
Fig. 5 ein Substrat mit den Kontaktflächen und der Kontaktzone,
Fig. 6 die zurückgesetzten Kontaktflächen im Substrat,
Fig. 7 Herstellungsschritte der Kontaktflächen im Substrat,
Fig. 8 Beispiele für Oberflächen-Strukturen der Kontaktflächen,
Fig. 9 das Design der Kontaktzone und der Kontaktflächen.

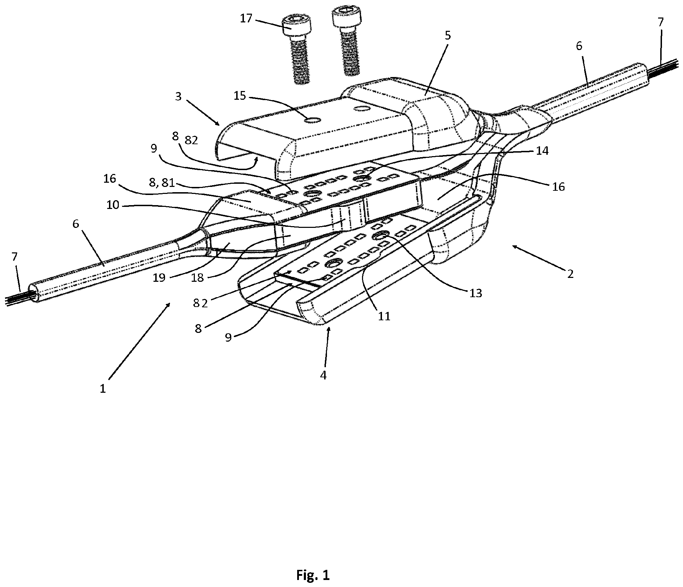

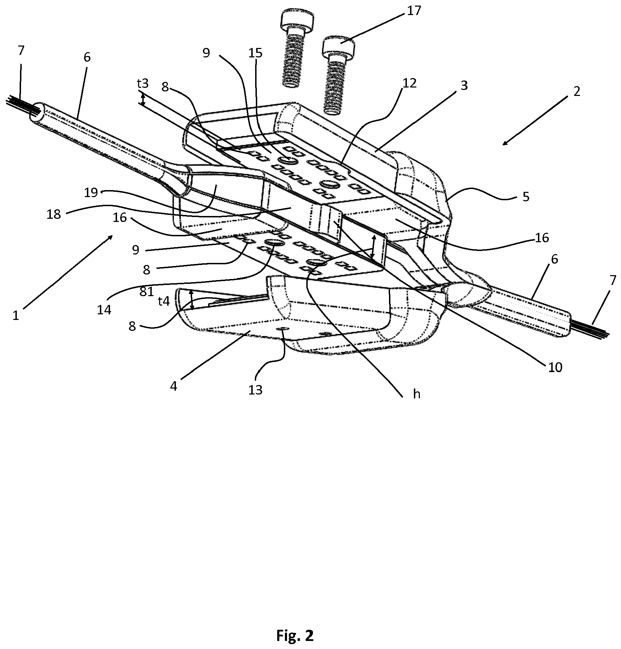

Fig. 1 bis Fig. 4 zeigen ein Ausführungsbeispiel des implantierbaren Steckverbinders gemäß der Erfindung. Der implantierbare Steckverbinder weist auf: einen Innenabschnitt 1, umfassend eine Anzahl von Kontaktflächen 9, die in zumindest einer Oberfläche 81 eines ersten Substrats 8 eingebettet sind, einen Außenabschnitt 2, umfassend eine Anzahl von Kontaktflächen 9, die in zumindest einer Oberfläche 82 eines zweiten Substrats 8 eingebettet sind, wobei der Außenabschnitt einen Raum definiert, in welchem der Innenabschnitt in einem Montage-Zustand aufnehmbar ist, wobei die Kontaktflächen 9 in einem Vormontage-Zustand im Verhältnis zu der jeweiligen Oberfläche 81, 82 des jeweiligen Substrats 8 zurückgesetzt sind, und wobei in einem Montage-Zustand der Innenabschnitt 1 gegen den Außenabschnitt 2 gepresst ist, derart, dass zueinander korrespondierende Kontaktflächen 9 miteinander in Berührung kommen.

Der Innenabschnitt 1 beinhaltet also das erste Substrat 8, und der Außenabschnitt 2 beinhaltet also das zweite Substrat 8.

Unter "Vormontage-Zustand" wird der Zustand am Ende des Herstellungsprozesses des Innenabschnitts und des Außenabschnitts verstanden. Unter "Montage-Zustand" werden die Verhältnisse nach Einführen des Innenabschnitts in den Außenabschnitt und Fixieren des Innenabschnitts im Außenabschnitt verstanden. Dann berühren sich paarweise einander entsprechende Kontaktflächen 9, d.h. der Steckverbinder ist elektrisch durchkontaktiert.

Der Außenabschnitt 2 umfasst zwei Gehäuseteile 3, 4. An den Enden des Innenabschnitts 1 und des Außenabschnitts 2 sind Kabel 6 angeordnet, die ihrerseits Leitungen 7 enthalten. Die Kabel 6 sind über Kontaktzonen 16, die an den Enden des Innenabschnitts 1 und des Außenabschnitts 2 angeordnet sind, mit den jeweiligen Kontaktflächen 9 verbunden. Die beiden Gehäuseteile 3, 4 des Außenabschnitts 2 umfassen jeweils eine Zahl von Kontaktflächen 9 in jeweils einer Oberfläche 82.

Der Steckverbinder dieses Ausführungsbeispiels ist 32-polig ausgestaltet. Um Platz zu sparen, sind die Kontaktflächen 9 des Außenabschnitts 2 auf zwei Oberflächen 82 mit jeweils Kontaktflächen 9 verteilt, wobei die 16 Kontaktflächen jeder Oberfläche 82 nochmals in 2 Reihen à 8 Kontaktflächen 9 aufgeteilt liegen. Nach Schließen der Steckverbindung, also im Montage-Zustand, greifen zwei "Arme" (also die Gehäuseteile 3, 4) des Außenabschnitts 2 um einen doppelseitig (also in der Figur oben und unten) mit Kontaktflächen 9 bestückten Innenabschnitt 1.

Der elektrische Kontakt wird über die Kontaktflächen 9 (planare Pads) hergestellt. Dabei handelt es sich um in das Substrat 8 aus Silikon eingebettete Platin-Iridium-Strukturen. Die so angeordneten Kontaktflächen (Pads) 9 haben den Vorteil, dass sie während der Operation leichter rückstandsarm zu reinigen sind als beispielsweise Pins und Buchsen.

Ein weiterer Vorteil dieses Aufbaus ist, dass zur Herstellung Fertigungsprozesse für implantierbare Elektroden eingesetzt werden können.

Der Verschlussmechanismus 13, 17 umfasst eine oder mehrere Schrauben 17, die jeweils durch ein Durchgangsloch 15 im einen Gehäuseteil 3 und durch ein Durchgangsloch 14 im Innenabschnitt in ein Gewindeloch 13 des anderen Gehäuseteils 4 gedreht werden. Die Schraubverbindung bietet die Möglichkeit, über die Definition von Drehmomenten die für einen zuverlässigen Kontakt nötige Anpresskraft zu garantieren. Die Schrauben 17 können vom Operateur mittels mitgelieferter Einweg-Drehmomentschraubendreher angezogen werden und stellen die mechanische (und damit auch die elektrische) Verbindung sicher.

Die Silikonsubstrate (Silikonmatten) 8, in welchen sich Leiterbahnen und Kontaktflächen (Pads) 9 befinden, liegen bei dem Außenabschnitt 2 in Wannen eingebettet, die durch die Gehäuseteile 3, 4 gebildet werden. Die Wannen bzw. die Gehäuseteile 3, 4 sind aus Metall gefertigt, vorzugsweise wird hierfür Titan verwendet. Der Außenabschnitt 2 ist mechanisch steif ausgebildet, damit die über die Schrauben 17 aufgebrachte Kraft gleichmäßig über die Oberflächen 82 mit den Kontaktflächen 9 verteilt wird und etwaige Verbiegungen minimiert werden.

Aufgrund der Kapselung mit dem Außenabschnitt 2 kann beim Innenabschnitt 1 auf die Verwendung von Metall als Trägermaterial verzichtet werden. Es kann Polyetheteretherketon (PEEK) oder z.B. jeder andere biostabile Kunststoff hoher Festigkeit verwendet werden.

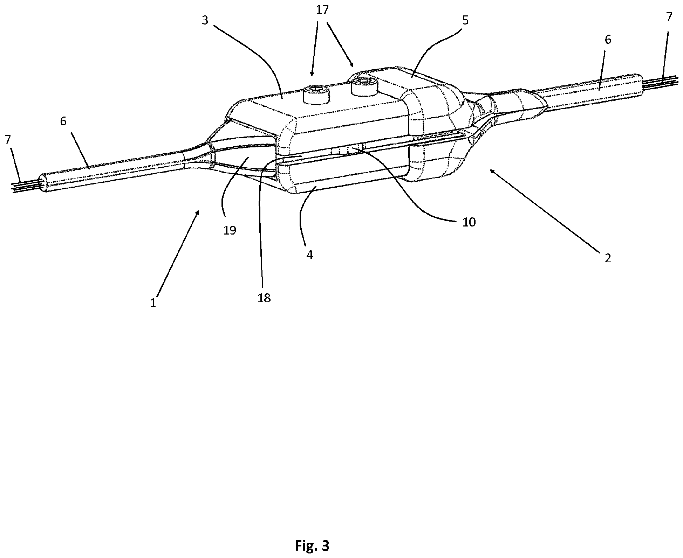

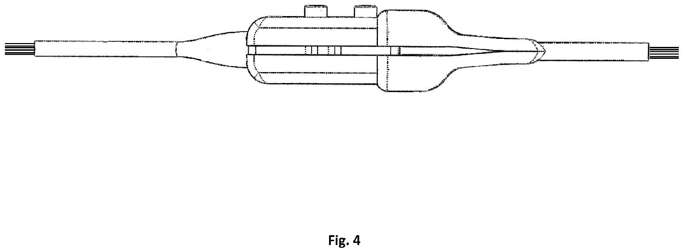

Der Innenabschnitt 1 hat eine etwas größere Höhe h als die Summe der Tiefen t3+ t4 der beiden Gehäuseteile 3, 4 des Außenabschnitts 2 und wird somit beim Verschluss (im Montage-Zustand) nicht vollständig umschlossen, vgl. Fig. 3 und Fig. 4. Es verbleibt ein kleiner Spalt zwischen den beiden Gehäuseteilen 3 und 4. Damit wird sichergestellt, dass die Anpresskraft auch tatsächlich auf die Substrate 8 mit den Kontaktflächen 9 wirkt und nicht etwa von den Rändern der Gehäuseteile 3, 4 abgefangen wird.

Im Ausführungsbeispiel umfasst jedes Gehäuseteil 3, 4 jeweils ein zweites Substrat 8 mit Kontaktflächen 9 in der jeweiligen Oberfläche 82. Demgemäß umfasst der Innenabschnitt auf zwei Flächen hierzu korrespondierende Kontaktflächen 9 in jeweils einer Oberfläche 81 eines ersten Substrats 8.

Die Kontaktflächen 9 müssen aber nicht jeweils auf beide Oberflächen 81 des Innenabschnitts 1 verteilt sein, die Erfindung umfasst auch den Fall, dass der Innenabschnitt 1 lediglich auf der einen Seite, z.B. auf der Oberseite, Kontaktflächen 9 enthält und dementsprechend auch der Außenabschnitt 2 lediglich an der Oberfläche des entsprechenden Gehäuseteils 3 oder 4 Kontaktflächen 9 enthält.

Der Übergang von den Kabeln 6 zum Steckverbinder ist durch Vergießen in Silikon ausgestaltet. Der empfindliche Anschlussbereich (die Kontaktzone) 16 wird so mechanisch entlastet und die elektrischen Kontaktierungen werden unterstützt und nach außen isoliert. Die Gehäuseteile 3, 4 sind so mit Silikon vergossen, dass sie leichter die Positionen des Montage-Zustands (also im geschlossenen Zustand) annehmen. Dieser Silikonverguss 5 erleichtert das Verschrauben des Systems. Die elektrische Verbindung der Drähte an die Anschlüsse des Steckverbinders ist durch Laserschweißen ausgeführt. In ähnlicher Weise gibt es einen Silikonverguss 19 der Silikonmatten miteinander und mit dem Substrat des Innenabschnitts 1.

Eine asymmetrische Form des Innenabschnitts 1 und der Gegenformen des Außenabschnitts 2 als Verpolungssicherungsmittel 10 gewährleistet die Verpolungssicherheit des Steckverbinders. Die Verpolungssicherungsmittel 10, 11, 12 umfassen im Ausführungsbeispiel eine Einbuchtung 11, 12 im einen Abschnitt sowie eine dazu korrespondierende Ausbuchtung 10 im anderen Abschnitt.

Die Kanten an der Stirnseite des Innenteils 1 sind mit Fasen versehen, wodurch die Gehäuseteile 3, 4 beim Einführen des Innenabschnitts 1 auseinandergedrückt werden.

Da es sich bei dem Steckverbinder gemäß der Erfindung um ein "In-line"-Design handelt, die Oberflächen 81, 82 mit den Kontaktflächen 9 also entlang der Längsachse des Steckverbinders orientiert sind, ist es auch kompatibel mit Führungsdrähten. Der hierzu vorgesehene Kanal durch den Innenabschnitt 1 kreuzt die Schraublöcher 14, somit ist eine Insertion eines Führungsdrahts nur bei nicht verschraubtem Steckverbinder möglich. Dies bietet die Möglichkeit, Fehlern beim Ablauf der Operationsprozedur vorzubeugen.

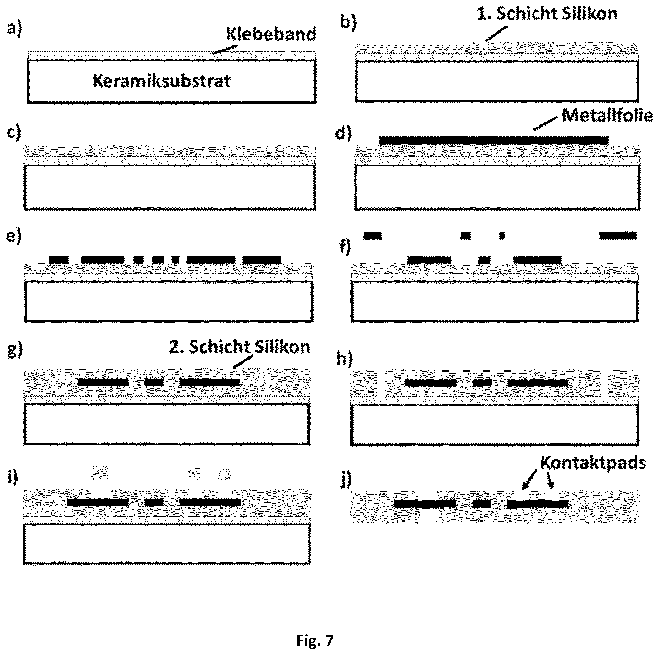

Fig. 7 zeigt die Prozessschritte zur Herstellung der Kontaktflächen. Hierbei werden im Wechsel Silikon und Metallfolien aufeinandergeschichtet und per Laser strukturiert. Die Herstellungsschritte der Kontaktflächen:
a-c) Aufschleudern und ggf. Strukturieren von Silikon;
d-f) Aufwalzen, Strukturieren und Abziehen von Metall;
g-i) Einbetten in Silikon und Öffnung der Kontakte;
j) Abheben des Schichtverbunds.

Im Einzelnen:
Zunächst wird ein Al₂O₃-Keramiksubstrat mit PVC-Klebeband laminiert (Fig. 7a). Darauf wird eine erste Schicht Silikon aufgeschleudert. Die Schichtdicke dieser Schicht kann von ca. 30 µm bei einer Drehzahl von 2000 rpm über 90 s bis hin zu ca. 70 µm bei 500 rpm über 90 s (Fig. 7b) betragen. Je nach Prozess wird die Silikonschicht noch mittels Laser geschnitten, um später das Metall auch von der Rückseite freilegen zu können (Fig. 7c). Diese beidseitige Kontaktierung ist nötig, falls die Anschlussdrähte per Laserschweißen an die Proben angeschlossen werden sollen, oder bei einer Faltung der Silikonmatten.

Im Anschluss wird Metallfolie aufgewalzt, per Laser strukturiert, und die Bereiche werden zwischen den Leiterbahnen abgezogen (Fig. 7d-f).

Eine zweite, ca. 32 µm dicke Schicht Silikon wird mit 4000 rpm über 90 s aufgeschleudert und ausgehärtet (Fig. 7g). Die Kontaktbereiche werden freigelegt, indem deren Umrisse mittels Laser ins Silikon geschnitten und das überschüssige Silikon mit einer Pinzette herausgezupft wird (Fig. 7h, i).

In einem letzten Laserschritt wird die Umrandung des Kontaktpad-Arrays ausgeschnitten. Der fertige Schichtverbund wird dann, ggf. unter Zugabe von Ethanol, vom Substrat abgelöst (Fig. 7j).

Um die Kontaktflächen 9 von Rückständen zu befreien, werden die Proben mit Ethanol und DI-Wasser gereinigt.

Um das Silikon per Rotationsbeschichtung aufbringen zu können, wird es zuvor im Verhältnis 1:1 mit n-Heptan verdünnt.

Als Material für die Kontaktflächen 9 kann PtIr-Folie (90 % Platin, 10% Iridium), 25 µm dick, verwendet werden.

Vorteilhafterweise wird ein Laser mit Pulsweiten im Pikosekundenbereich eingesetzt ("Pikosekundenlaser"). Pulsweiten im Pikosekundenbereich ermöglichen eine präzisere und selektivere Strukturierung als z.B. Pulsweiten im Nanosekundenbereich ("Nanosekundenlaser"). Als Laser kann ein Nd:YVO4-Laser eingesetzt werden.

Ein weiterer Vorteil des Pikosekundenlasers ist die höhere Reproduzierbarkeit, was das Ausrichten der Oberflächen betrifft. Mehrfaches Herausnehmen und wieder Einlegen führt beim Nanosekundenlaser oft zu einem Versatz zwischen den gelaserten Strukturen.

Überraschenderweise hat sich herausgestellt, dass die Auswahl des Lasers zur Strukturierung der Oberfläche der Kontaktflächen 9 einen Einfluss auf die Ausbildung des elektrischen Kontakts hat.

Vorteilhafterweise werden die Kontaktflächen (Fig. 7d-f) also mit dem Pikosekundenlaser aus Ptlr hergestellt. Im Anschluss werden die freiliegenden Metalloberflächen mit dem Nanosekundenlaser strukturiert. Ziel dieses zusätzlichen Schritts ist die gezielte Generation von Materialaufwürfen durch Aufschmelzen. Derartige Erhöhungen der sonst flachen Kontaktflächen tragen zur Kontaktbildung bei.

Das Muster für diese Strukturierung bzw. die Dichte der Materialaufwürfe beeinflusst also die Kontaktbildung. Hierfür kann die Anzahl der Schnitte mit dem Nanosekundenlaser variiert werden. Die Schnitte werden vorteilhafterweise so diagonal ausgerichtet, dass sie sich beim Aufeinanderlegen (also im Montage-Zustand) der Kontaktflächen 9 kreuzen. Vorteilhafterweise werden mehrere Schnitte (z.B. jeweils sieben Schnitte) pro Kontaktfläche aufgebracht.

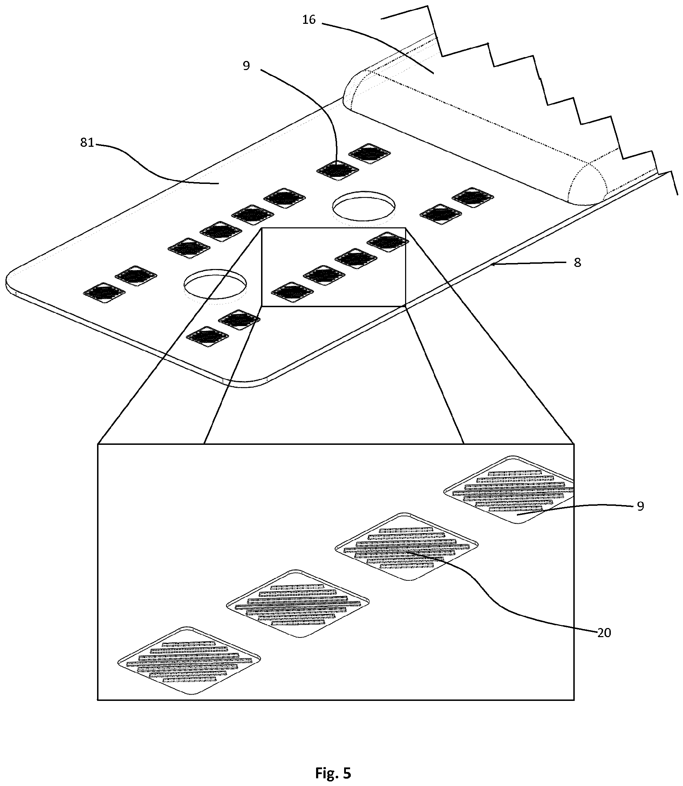

Fig. 5 zeigt das Substrat 81, 82 mit den Kontaktflächen 9. Die Kontaktflächen sind mit dreidimensionalen Strukturen 20 versehen. Diese Strukturen 20 sind als diagonale Furchen ausgebildet, die sich beim Berühren der jeweiligen Kontaktflächen 9 kreuzen.

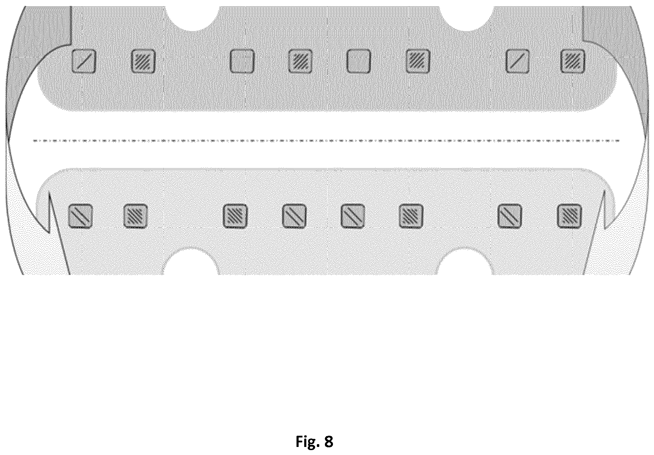

Fig. 8 zeigt weitere mögliche Muster für die dreidimensionalen Strukturen.

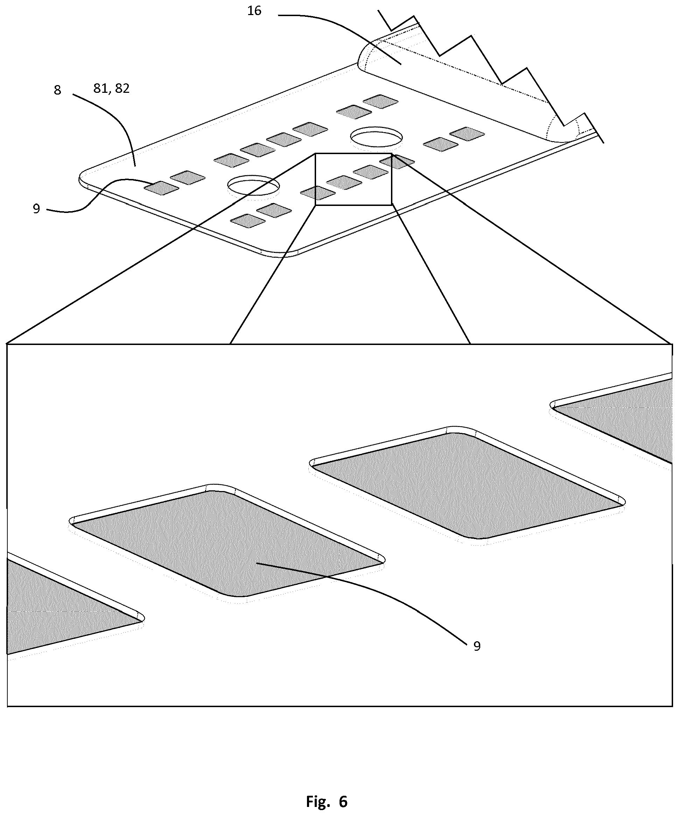

Fig. 6 zeigt die Anordnung der Kontaktflächen 9 im Verhältnis zu den Oberflächen 81, 82. Wie dort sichtbar ist, sind die Kontaktflächen 9 in die Oberflächen eingebettet, liegen zurückgesetzt, also etwas tiefer als die Oberflächen 81, 82. Die Kontaktflächen liegen etwa um die Dicke der zweiten Silikonschicht (im Ausführungsbeispiel (etwa 32 µm) tiefer.

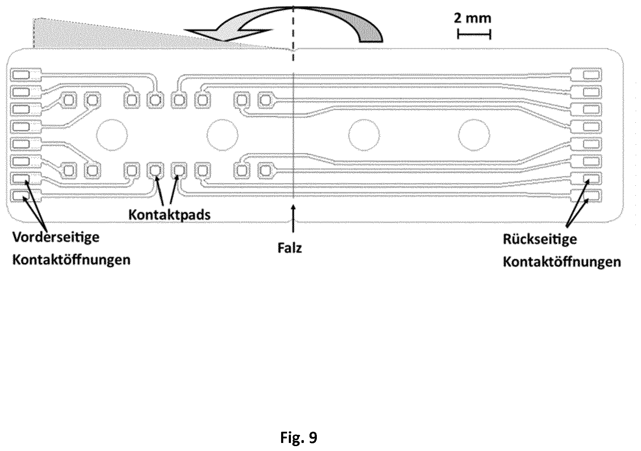

Die Kontaktöffnungen an der Kontaktzone 16 und die Leiterbahnen zu den Kontaktflächen 9 sind ein limitierender Faktor für die Anzahl der auf dem Substrat realisierbaren Kanäle. Um die Anzahl der Kontaktflächen 9 erhöhen zu können, kann das Silikonsubstrat zusammen mit den Kontaktflächen 9 (die Silikonmatten) 8 einmal gefaltet werden. Dies erhöht die Leiterbahndichte und dient dazu, die Abmessungen des Steckverbinders zu minimieren. Fig. 9 stellt dies dar. Um die gefalteten Silikonmatten miteinander zu verbinden, werden deren Oberflächen vor dem Zusammenfügen plasmaaktiviert.

Als Kabel 6 werden 60 µm dicke Drähte 7, geführt durch einen 1,8 mm dicken Silikonschlauch, zum Einsatz. Für den Anschluss der beiden Außenteile des Steckverbinders wird ein Kabel, welches sich von 1 x 32 Kanälen auf 2 x 16 Kanäle aufteilt, eingesetzt.

Die Kabel 6 können an die Kontaktstellen des Silikonsubstrats (der Silikonmatten) 8 angelötet und die Lötstellen mit Silikon vergossen werden.

Mittels Silikonkleber können die Kontaktflächen-Substrate 81, 82 auf den Gehäuseteilen fixiert werden. Im Anschluss daran kann damit auch der gesamte Übergangsbereich von Kabel zu Stecker mithilfe von Teflon-Formen vergossen werden. Um die Blasenbildung zu minimieren, kann das Silikon unter einem Überdruck von ca. 1,2 bar bei 100 °C ausgehärtet werden.

In Montage-Zustand werden die beiden "Arme", also die beiden Gehäuseteile 3, 4, des Steckverbinders zusammengefügt. Hierfür wird der Steckverbinder zunächst geschlossen und mit Schrauben fixiert, damit die Außenteile nach dem Verkleben den geschlossenen Zustand bevorzugt einnehmen.

Durch das Zusammenfügen der beiden Gehäuseteile 3, 4 wird das Innenteil 1, also das Silikonsubstrat 8, gegen die Gehäuseteile bzw. die darin angeordneten Substrate, gepresst. Weil die Höhe h1 des Innenteils etwas größer ist als die Summe der Tiefen t3, t4 der Gehäuseteile und die Silikonsubstrate 8 dem Raum nicht entweichen können, pressen die Substrate 8 die zurückgesetzten Kontaktflächen 9 in den jeweils darüber liegenden Luftraum, der sich leicht komprimieren lässt, bis sich einander korrespondierende (gegenüberliegende) Kontaktflächen 9 paarweise miteinander in Berührung kommen.

Weil das Silikonsubstrat flexibel ist, ist dieser Vorgang beliebig oft reversibel. Der Steckverbinder lässt sich durch Lösen der Schraubenverbindung 17 beliebig oft wieder öffnen, die mechanische und elektrische Verbindung also trennen, und von Neuem wieder verschließen.

### Bezugszeichenliste

- 1: Innenabschnitt
- 2: Außenabschnitt
- 3, 4: Gehäuseteil
- 5: Silikonverguss der Außenschalen und Silikonmatte
- 6: Mehrkanal-Kabel
- 7: Leiter/Drähte/Adern
- 8: erstes Substrat, zweites Substrat (Polymer/Gummi-Matte)
- 9: Kontaktflächen, Kontaktpads
- 10: Ausbuchtung als Verpolungssicherungsmittel
- 11, 12: Einbuchtung/Aussparung als Verpolungssicherungsmittel
- 13: Gewindeloch für Schraube
- 14, 15: Durchgangsloch für Schraube
- 16: Kontaktzone/ Anschlüsse an Kabel
- 17: Schraube
- 18: Träger Innenteil
- 19: Silikonverguss von Silikonmatten miteinander und mit Substrat von Innenteil
- 20: 3D-Strukurierung der Kontaktfläche
- 81: Oberfläche des ersten Substrats
- 82: Oberfläche des zweiten Substrats
- h: Höhe des Innenteils 1
- t3: Wannentiefe des ersten Gehäuseteils 3
- t4: Wannentiefe des zweiten Gehäuseteils 4

## Patentansprüche

1. Implantierbarer Steckverbinder, aufweisend:
einen Innenabschnitt (1), umfassend eine Anzahl von Kontaktflächen (9), die in zumindest einer Oberfläche (81) eines ersten Substrats (8) eingebettet sind,
einen Außenabschnitt (2), umfassend eine Anzahl von Kontaktflächen (9), die in zumindest einer Oberfläche (82) eines zweiten Substrats (8) eingebettet sind,
wobei der Außenabschnitt (2) einen Raum definiert, in welchem der Innenabschnitt (1) in einem Montage-Zustand aufnehmbar ist, **dadurch gekennzeichnet, dass**
die Kontaktflächen (9) sowohl des Innenabschnitts (1) als auch des Außenabschnitts (2) in einem Vormontage-Zustand im Verhältnis zu der Oberfläche (81, 82) des jeweiligen Substrats (8) zurückgesetzt sind,
und wobei der Steckverbinder derart ausgebildet ist, dass der Innenabschnitt (1) in einem Montage-Zustand gegen den Außenabschnitt (2) gepresst ist, derart, dass zueinander korrespondierende Kontaktflächen (9) miteinander in Berührung kommen.

2. Implantierbarer Steckverbinder gemäß Anspruch 1, wobei die Kontaktflächen (9) zumindest stellenweise strukturiert sind, insbesondere konvex.

3. Implantierbarer Steckverbinder gemäß einem der vorherigen Ansprüche, wobei im Montage-Zustand auf einer Oberfläche (81, 82) benachbarte Kontaktflächen (9) gegeneinander elektrisch isoliert sind.

4. Implantierbarer Steckverbinder gemäß einem der vorherigen Ansprüche, wobei im Montage-Zustand die Kontaktflächen (9) gegen den Raum abgedichtet sind.

5. Implantierbarer Steckverbinder gemäß einem der vorherigen Ansprüche, wobei der Außenabschnitt (2) zumindest zwei Gehäuseteile (3, 4) mit jeweils vorbestimmten Tiefen (t3, t4) umfasst, die im Montage-Zustand den Raum definieren.

6. Implantierbarer Steckverbinder gemäß Anspruch 5, wobei jeder der Gehäuseteile (3, 4) jeweils eine Zahl von Kontaktflächen (9) in jeweils einer Oberfläche (82) umfassen.

7. Implantierbarer Steckverbinder gemäß einem der vorherigen Ansprüche, wobei im Vormontage-Zustand die Summe der Tiefen (t3, t4) der Gehäuseteile geringer als die Höhe (h1) des Innenabschnitts (1) ist.

8. Implantierbarer Steckverbinder gemäß einem der vorherigen Ansprüche, weiter aufweisend: eine Schraubverbindung (13, 17), mit welcher im Montage-Zustand der Innenabschnitt (1) gegen den Außenabschnitt (2) gepresst ist.

9. Implantierbarer Steckverbinder gemäß einem der vorherigen Ansprüche, weiter aufweisend: einen Kanal in Axialrichtung, der ein Einführen eines Führungsdrahts ermöglicht.

10. Implantierbarer Steckverbinder gemäß dem vorherigen Anspruch, wobei der Kanal im Montage-Zustand durch die Schraubverbindung (13, 17) blockiert ist.

11. Implantierbarer Steckverbinder gemäß einem der vorherigen Ansprüche, weiter aufweisend: Kontaktzonen (16), die einerseits mit Leitungen (7), andererseits mit den Kontaktflächen (9) verbindbar sind.

12. Implantierbarer Steckverbinder gemäß einem der vorherigen Ansprüche, weiter aufweisend: Verpolungssicherungsmittel (10, 11, 12), welche gewährleisten, dass im Montage-Zustand der Innenabschnitt in genau einer Orientierung bezüglich des Außenabschnitts in dem Außenabschnitt aufnehmbar ist.

13. Implantierbarer Steckverbinder gemäß einem der vorherigen Ansprüche, wobei der Außenabschnitt (2) und/oder der Innenabschnitt (1) zumindest teilweise mit Silikon vergossen sind.

14. Verfahren zum Herstellen von Kontaktflächen für einen Steckverbinder gemäß einem der vorherigen Ansprüche, umfassend die Schritte:
- Aufschleudern einer ersten Silikonschicht auf ein Substrat;
- Aufwalzen von Metall;
- Strukturieren des aufgewalzten Metalls zur Bildung von Leiterbahnen;
- Aufschleudern einer zweiten Silikonschicht;
- Freilegen der Kontaktflächen (9).

15. Verfahren gemäß dem vorherigen Anspruch, wobei das Strukturieren mittels eines Pikosenkundenlasers durchgeführt wird.

16. Verfahren gemäß Anspruch 14 oder 15, wobei die freigelegten Kontaktflächen (9) strukturiert werden, vorzugsweise mittels eines Nanoseklundenlasers.

## Claims

1. An implantable connector comprising:
an inner portion (1) including a number of contact surfaces (9) which are embedded in at least one surface (81) of a first substrate (8),
an outer portion (2) including a number of contact surfaces (9) which are embedded in at least one surface (82) of a second substrate (8),
said outer portion (2) defining an area within which said inner portion (1) may be received when in an assembled state,
**characterised in that** in a state prior to assembly, the contact surfaces (9) of both the inner portion (1) and the outer portion (2) are recessed with respect to the surfaces (81, 82) of the respective substrates (8), and **in that** the connector is designed in such a manner that in an assembled state, the inner portion (1) is pressed against the outer portion (2) so that the mutually corresponding contact surfaces (9) are brought into contact with each other.

2. The implantable connector as claimed in claim 1, wherein the contact surfaces (9) are structured at least at certain points, particularly in a convex manner.

3. The implantable connector as claimed in any of the preceding claims, wherein in an assembled state, contact surfaces (9) located adjacent to each other on a surface (81, 82) are electrically insulated against each other.

4. The implantable connector as claimed in any of the preceding claims, wherein in an assembled state, the contact surfaces (9) are sealed against said area.

5. The implantable connector as claimed in any of the preceding claims, wherein the outer portion (2) comprises at least two housing parts (3, 4) having each predetermined depths (t3, t4) which define said area when in an assembled state.

6. The implantable connector as claimed in claim 5, wherein each of the housing parts (3, 4) comprises a number of contact surfaces (9) located respectively in one surface (82).

7. The implantable connector as claimed in any of the preceding claims, wherein in a state prior to assembly, the sum of the depths (t3, t4) of the housing parts is smaller than the height (h1) of the inner portion (1).

8. The implantable connector as claimed in any of the preceding claims, further comprising: a threaded connection (13, 17) by means of which the inner portion (1) is pressed against the outer portion (2) when in an assembled state.

9. The implantable connector as claimed in any of the preceding claims, further including: a channel formed in an axial direction which permits a guide wire to be introduced therein.

10. The implantable connector as claimed in the preceding claim, wherein in an assembled state, the channel is blocked by said threaded connection (13, 17).

11. The implantable connector as claimed in any of the preceding claims, further including: contact zones (16) which are connectible with leads (7), on the one hand, and with the contact surfaces (9), on the other.

12. The implantable connector as claimed in any of the preceding claims, further including: reverse polarity protection means (10, 11, 12) which make sure that in the assembled state, the inner portion may be received within the outer portion in exactly one orientation relative to said outer portion.

13. The implantable connector as claimed in any of the preceding claims, wherein the outer portion (2) and/or the inner portion (1) is/are at least partially potted in silicone.

14. A method of fabricating contact surfaces for a connector as claimed in any of the preceding claims, comprising the steps of:
- depositing a first silicone layer onto a substrate by spin coating;
- applying metal by roll-coating;
- structuring the rolled-on metal for forming strip conductors;
- depositing a second silicone layer by spin coating;
- exposing the contact surfaces (9).

15. The method as claimed in the preceding claim, wherein the structuring is realised by means of a picosecond laser.

16. The method as claimed in claim 14 or 15, wherein the exposed contact surfaces (9) are structured, preferably by means of a nanosecond laser.

## Revendications

1. Connecteur implantable, lequel présente :
une partie intérieure (1) comprenant un nombre donné de surfaces de contact (9) qui sont intégrées dans au moins une surface (81) d'un premier substrat (8),
une partie extérieure (2) comprenant un nombre donné de surfaces de contact (9) qui sont intégrées dans au moins une surface (82) d'un deuxième substrat (8),
la partie extérieure (2) définissant un espace dans lequel la partie intérieure (1) peut être reçue à l'état de montage,
**caractérisé en ce que** les surfaces de contact (9) de la partie intérieure (1) ainsi que de la partie extérieure (2) se trouvent, dans un état de prémontage, en retrait par rapport à la surface (81, 82) du substrat (8) respectif, le connecteur étant formé de telle sorte que, dans un état de montage, la partie intérieure (1) est pressée de manière telle contre la partie extérieure (2) que des surfaces de contact (9) qui se correspondent entrent en contact les unes avec les autres.

2. Connecteur implantable selon la revendication 1, dans lequel les surfaces de contact (9) sont structurées au moins par endroits, en particulier de manière convexe.

3. Connecteur implantable selon l'une quelconque des revendications précédentes, dans lequel, à l'état de montage, des surfaces de contact (9) voisines sur une surface (81, 82) sont électriquement isolées l'une de l'autre.

4. Connecteur implantable selon l'une quelconque des revendications précédentes, dans lequel, à l'état de montage, les surfaces de contact (9) sont rendues étanches par rapport audit espace.

5. Connecteur implantable selon l'une quelconque des revendications précédentes, dans lequel la partie extérieure (2) comprend au moins deux parties de boîtier (3, 4) qui présentent respectivement des profondeurs prédéterminées (t3, t4) et qui, à l'état de montage, définissent ledit espace.

6. Connecteur implantable selon la revendication 5, dans lequel chacune des parties de boîtier (3, 4) comprend respectivement un nombre donné de surfaces de contact (9) situées dans respectivement une surface (82).

7. Connecteur implantable selon l'une quelconque des revendications précédentes, dans lequel, à l'état de prémontage, la somme des profondeurs (t3, t4) des parties de boîtier est inférieure à la hauteur (h1) de la partie intérieure (1).

8. Connecteur implantable selon l'une quelconque des revendications précédentes, lequel présente en outre : un assemblage vissé (13, 17) grâce auquel, à l'état de montage, la partie intérieure (1) est pressée contre la partie extérieure (2).

9. Connecteur implantable selon l'une quelconque des revendications précédentes, lequel présente en outre : un canal en sens axial qui permet l'insertion d'un fil de guidage.

10. Connecteur implantable selon la revendication précédente, dans lequel le canal est bloqué, à l'état de montage, par l'assemblage vissé (13, 17).

11. Connecteur implantable selon l'une quelconque des revendications précédentes, lequel présente en outre : des zones de contact (16) qui peuvent être connectées d'un côté avec des fils électriques (7), d'un autre côté avec les surfaces de contact (9).

12. Connecteur implantable selon l'une quelconque des revendications précédentes, lequel présente en outre : des moyens de protection contre les erreurs de polarisation (10, 11, 12) lesquels garantissent que la partie intérieure peut, à l'état de montage, être reçue dans la partie extérieure, et ce uniquement dans une orientation donnée par rapport à ladite partie extérieure.

13. Connecteur implantable selon l'une quelconque des revendications précédentes, dans lequel la partie extérieure (2) et/ou la partie intérieure (1) sont coulées au moins en partie dans de la silicone.

14. Procédé destiné à fabriquer des surfaces de contact pour un connecteur selon l'une quelconque des revendications précédentes, lequel comprend les étapes suivantes :
- l'application par centrifugation d'une première couche de silicone sur un substrat ;
- le placage de métal ;
- la structuration du métal plaqué en vue de former des pistes conductrices ;
- l'application par centrifugation d'une deuxième couche de silicone ;
- la mise à nu des surfaces de contact (9).

15. Procédé selon la revendication précédente, lors duquel la structuration est réalisée au moyen d'un laser picoseconde.

16. Procédé selon la revendication 14 ou 15, lors duquel les surfaces de contact (9) mises à nu sont structurées, de préférence au moyen d'un laser nanoseconde.
